# EUROPÄISCHE PATENTSCHRIFT

(11) **EP 3 409 675 B1**
(45) Veröffentlichungstag und Bekanntmachung des Hinweises auf die Patenterteilung: **22.01.2020**
(21) Anmeldenummer: 18174669.4
(22) Anmeldetag: 29.05.2018
(51) Int. Cl.: C07D 498/14, C08G 18/79

(54) **POLYCYCLISCHE DIIMINOOXADIAZINONE**
POLYCYCLIC DIIMINOOXADIAZINONES
DI-IMINO-OXADIAZINONE POLYCYCLIQUE

(30) Priorität: 30.05.2017 EP 17173345; 09.06.2017 LU 100278
(43) Veröffentlichungstag der Anmeldung: 05.12.2018
(73) Patentinhaber: Covestro Deutschland AG, 51373 Leverkusen (DE)
(72) Erfinder: RICHTER, Frank, 51373 Leverkusen (DE); ORECCHIA, Patrizio, 13349 Berlin (DE); WIDEMANN, Max, 72221 Haiterbach-Beihingen (DE)
(74) Vertreter: Levpat

(56) Entgegenhaltungen:
- EP-A1- 0 849 268
- EP-A2- 0 761 670
- EP-A2- 0 841 088

## Beschreibung

Die Erfindung betrifft polycyclische Diiminooxadiazinone, Verfahren zu deren Herstellung sowie die Verwendung der polycyclischen Diiminooxadiazinone als Reaktivkomponente und die aus den polycyclischen Diiminooxadiazinonen erhältlichen Umsetzungsprodukte.

Die Substanzklasse der Diiminooxadiazinone A ist bisher gänzlich unbekannt, was - ohne an wissenschaftliche Theorien gebunden sein zu wollen - in der deutlich höheren Stabilität der isomeren Iminotriazindione B begründet sein könnte. Letztere können sich bei der Modifizierung von Isocyanaten in wechselnden Anteilen bilden und sind beispielsweise aus US 2007/0043152 A1 bekannt.

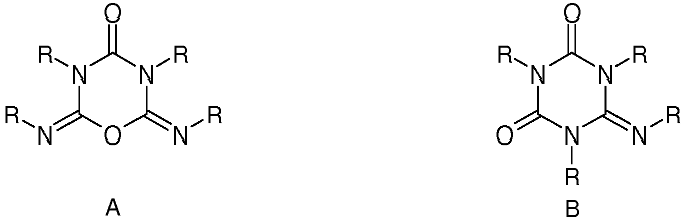

Monocyclische als auch polycyclische Verbindungen mit Iminooxadiazindionstruktur sind von EP0761670, EP0849268 und EP0841088 bekannt.

Der Erfindung lag die Aufgabe zugrunde, potenzielle Vernetzer für die Polyurethanchemie bereitzustellen, die keine freien, hochreaktiven Isocyanatgruppen enthalten und trotzdem eine Reaktivität gegenüber Verbindungen aufweisen, die Zerevitinov-aktive Wasserstoffatome enthalten.

Diese Aufgabe wurde erfindungsgemäß gelöst durch polycyclische Diiminooxadiazinone der Formel I und/oder der Formel II, 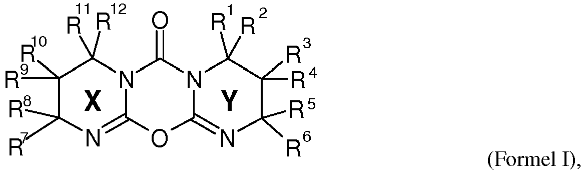 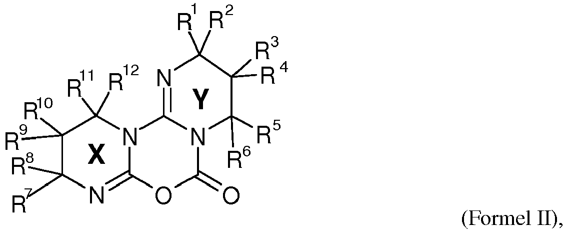 in welchen jeweils
- R¹ bis R¹²: unabhängig voneinander für Wasserstoff, einen gesättigten oder ungesättigten, linearen oder verzweigten, aliphatischen oder cycloaliphatischen organischen Rest mit 1 bis 20 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 20 Kohlenstoffatomen, der unsubstituiert oder mit gegenüber Isocyanatgruppen inerten Resten substituiert ist, stehen, wobei zwei oder mehrere der oben genannten Reste R¹ bis R⁶ jeweils miteinander und unter Einbeziehung eines, zweier oder dreier Kohlenstoffatome des annelierten Ringsegmentes Y weitere Ringe bilden können und/oder zwei oder mehrere der oben genannten Reste R⁷ bis R¹² jeweils miteinander und unter Einbeziehung eines, zweier oder dreier Kohlenstoffatome des annelierten Ringsegmentes X weitere Ringe bilden können.

Die gegebenenfalls in weitere Ringe einbezogenen ein, zwei oder drei Kohlenstoffatome der annelierten Ringsegmente X und Y sind vorliegend die sp³-hybridisierten Kohlenstoffatome der annelierten Ringsegmente X und Y.

Vorzugsweise sind die gegebenenfalls in weitere Ringe einbezogenen ein, zwei oder drei Kohlenstoffatome der annelierten Ringsegmente X und Y diejenigen Kohlenstoffatome, an denen der entsprechende ringbildende Rest R¹ bis R¹² befestigt ist.

Als gegenüber Isocyanatgruppen inerte Reste werden im Sinne der vorliegenden Erfindung alle Substituenten verstanden, die keine Zerevitinoff-aktiven Wasserstoffatome tragen.

Ein "Zerevitinoff-reaktives Wasserstoffatom" wird im Rahmen der vorliegenden Erfindung als ein azides Wasserstoffatom oder "aktives" Wasserstoffatom definiert. Ein solches wird in an sich bekannter Weise durch eine Reaktivität mit einem entsprechenden Grignard-Reagenz ermittelt. Die Menge an Zerevitinoff-aktiven Wasserstoffatomen wird typischerweise über die Methanfreisetzung gemessen, die gemäß der folgenden Reaktionsgleichung bei einer Reaktion der zu überprüfenden Substanz R-XH (die Funktionalität -XH bindet an den Rest des Moleküls R) mit Methylmagnesiumbromid (CH₃-MgBr) erfolgt:

CH₃-MgBr + R-XH → CH₄+ Mg(XR)Br

Als gegenüber Isocyanatgruppen inerte Heteroatome in der Kette werden im Sinne der vorliegenden Erfindung insbesondere O, S und SiR', ferner NR', verstanden, bei denen die Substituenten R' wiederum gegenüber Isocyanatgruppen inerte Reste der oben stehenden Definition sind.

Bevorzugt bedeuten die Bezugnahmen auf "umfassend", "enthaltend" usw. "im Wesentlichen bestehend aus" und ganz besonders bevorzugt "bestehend aus".

In einer ersten bevorzugten Ausführungsform stehen R¹ bis R¹² unabhängig voneinander für Wasserstoff, einen gesättigten oder ungesättigten, linearen oder verzweigten, aliphatischen oder cycloaliphatischen organischen Rest mit 1 bis 10 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 12 Kohlenstoffatomen, der unsubstituiert oder mit gegenüber Isocyanatgruppen inerten Resten substituiert ist, wobei zwei oder mehrere der oben genannten Reste R¹ bis R⁶ jeweils miteinander und unter Einbeziehung eines, zweier oder dreier Kohlenstoffatome des annelierten Ringsegmentes Y weitere Ringe bilden können und/oder zwei oder mehrere der oben genannten Reste R⁷ bis R¹² jeweils miteinander und unter Einbeziehung eines, zweier oder dreier Kohlenstoffatome des annelierten Ringsegmentes X weitere Ringe bilden können.

Hierbei ist es weiter bevorzugt, dass R¹ bis R¹² unabhängig voneinander für Wasserstoff, einen gesättigten oder ungesättigten, linearen oder verzweigten, aliphatischen oder cycloaliphatischen organischen Rest mit 1 bis 6 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, stehen, wobei zwei oder mehrere der oben genannten Reste R¹ bis R⁶ jeweils miteinander und unter Einbeziehung eines, zweier oder dreier Kohlenstoffatome des annelierten Ringsegmentes Y weitere Ringe bilden können und/oder zwei oder mehrere der oben genannten Reste R⁷ bis R¹² jeweils miteinander und unter Einbeziehung eines, zweier oder dreier Kohlenstoffatome des annelierten Ringsegmentes X weitere Ringe bilden können.

In einer weiteren bevorzugten Ausführungsform stehen R¹ bis R¹² unabhängig voneinander für Wasserstoff oder C₁-C₁₀-Alkyl(en), bevorzugt unabhängig voneinander für Wasserstoff oder C₁-C₆-Alkyl(en) und besonders bevorzugt unabhängig voneinander für Wasserstoff oder C₁-C₄-Alkyl(en). In einer weiteren bevorzugten Ausführungsform sind die gegenüber Isocyanatgruppen inerten Heteroatome Sauerstoff, Schwefel, tertiärer Stickstoff und/oder quartäres Silizium, wobei die Substituenten am Stickstoff und Silizium gegenüber Isocyanatgruppen inert sind, bevorzugt Sauerstoff und/oder Schwefel sind und besonders bevorzugt Sauerstoff ist.

Bei den gegebenenfalls aus zwei oder mehreren der Reste R¹ bis R⁶ jeweils untereinander und unter Einbeziehung eines, zweier oder dreier Kohlenstoffatome des annelierten Ringsegmentes Y gebildeten Ringen und/oder gegebenenfalls aus zwei oder mehreren der Reste R⁷ bis R¹² jeweils untereinander und unter Einbeziehung eines, zweier oder dreier Kohlenstoffatome des annelierten Ringsegmentes X gebildeten Ringen, gilt die jeweils vorstehend genannte Definition der Reste R¹ bis R¹² sowie deren als bevorzugt genannte Ausführungsformen, wobei besonders bevorzugt ist, dass die gegebenenfalls gebildeten Ringe 5 bis 8 Kohlenstoffatomen im Ring, besonders bevorzugt 5 bis 6 Kohlenstoffatomen im Ring aufweisen, wobei diese Ringe Heteroatome ausgewählt aus der Gruppe bestehend aus Sauerstoff, Schwefel, tertiärer Stickstoff und/oder quartäres Silizium, wobei die Substituenten am Stickstoff und Silizium gegenüber Isocyanatgruppen inert sind, aufweisen können.

Gemäß einer weiteren bevorzugten Ausführungsform der erfindungsgemäßen polycyclischen Diiminooxadiazinone der Formel I und/oder der Formel II, bilden die Reste R² und R⁵ miteinander und unter Einbeziehung der drei sp³-hybridisierten Kohlenstoffatome des annelierten Ringsegments Y einen weiteren Ring sowie die Reste R⁸ und R¹¹ miteinander und unter Einbeziehung der drei sp³-hybridisierten Kohlenstoffatome des annelierten Ringsegments X einen weiteren Ring. Hierbei ist weiterhin bevorzugt, dass die erfindungsgemäßen polycyclischen Diiminooxadiazinone Verbindungen der Formel III und/oder Verbindungen der Formel IV umfassen, 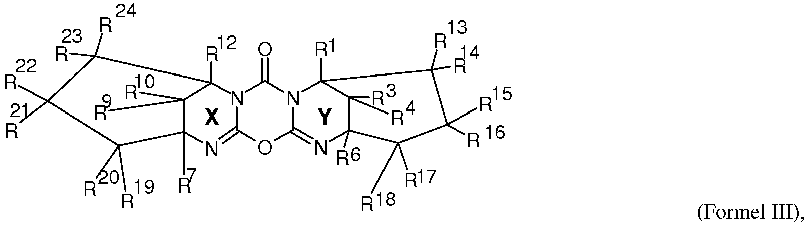 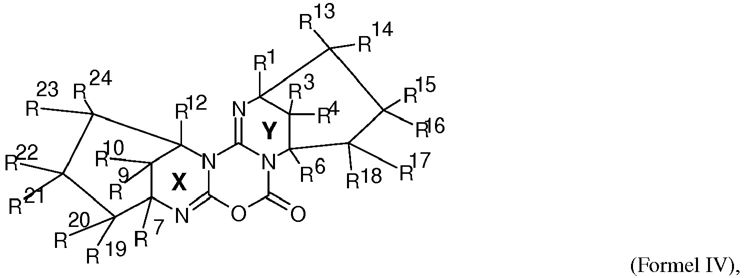 in welchen jeweils
- R¹, R³, R⁴, R⁶, R⁷, R⁹, R¹⁰ und R¹²: unabhängig voneinander für Wasserstoff, einen gesättigten oder ungesättigten, linearen oder verzweigten, aliphatischen oder cycloaliphatischen organischen Rest mit 1 bis 20 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 20 Kohlenstoffatomen, der unsubstituiert oder mit gegenüber Isocyanatgruppen inerten Resten substituiert ist, stehen und
- R¹³ bis R²⁴: unabhängig voneinander für Wasserstoff, einen gesättigten oder ungesättigten, linearen oder verzweigten, aliphatischen oder cycloaliphatischen organischen Rest mit 1 bis 20 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 20 Kohlenstoffatomen, der unsubstituiert oder mit gegenüber Isocyanatgruppen inerten Resten substituiert ist, stehen,
wobei zwei oder mehrere der oben genannten Reste R¹, R³, R⁴, R⁶, R¹³ bis R¹⁸ jeweils miteinander weitere Ringe bilden können und/oder zwei oder mehrere der oben genannten Reste R⁷, R⁹, R¹⁰, R¹², R¹⁹ bis R²⁴ jeweils miteinander weitere Ringe bilden können.

Ein weiterer Gegenstand der vorliegenden Erfindung ist ein Verfahren zur Herstellung der erfindungsgemäßen polycyclischen Diiminooxadiazinone, bei dem mindestens zwei Diisocyanate, bei denen die beiden NCO-Gruppen in 1,3-Position zueinander stehen, unter CO₂-Abspaltung cyclisiert werden. Diese Cyclisierung wird im Folgenden auch als Umsetzung bezeichnet und kann auch über zwei Stufen erfolgen.

In einer ersten bevorzugten Ausführungsform des erfindungsgemäßen Verfahrens werden die mindestens zwei Diisocyanate in einem ersten Schritt i) zu einem polycyclischen Iminooxadiazindion cyclisiert, welches
a) isoliert wird und in einem weiteren Schritt ii) zu dem polycyclischen Diiminooxadiazinon umgesetzt wird oder
b) ohne Isolierung in einem weiteren Schritt ii) direkt zu dem polycyclischen Diiminooxadiazinon umgesetzt wird.

In einer ebenfalls bevorzugten Ausführungsform erfolgt eine simultane CO₂-Abspaltung und Cyclisierung zu den erfindungsgemäßen polycyclischen Diiminooxadiazinonen. Hierfür geeignete Katalysatoren sind beispielsweise typische Carbodiimidisierungskatalysatoren wie z.B. in US 2 853 473 beschrieben.

Die mindestens bicyclischen Iminooxadiazindione sind auf Seite 2, Zeile 40 bis Seite 3, Zeile 25 der EP 0 761 670 A2 beschrieben.

Geeignete Diisocyanate bei denen die beiden NCO-Gruppen in 1,3-Position zueinander stehen liegen bevorzugt im Molekulargewichtsbereich von 126 bis 500 g/mol, besonders bevorzugt von 126 bis 350 g/mol. Beispiele für derartige 1,3-Diisocyanate sind 1,3-Propandiisocyanat, 1,3-Butandiisocyanat, 1,3-Pentandiisocyanat, 2,4-Pentandiisocyanat und andere Isomere der vorstehend genannten Diisocyanate mit 1,3-Stellung der NCO-Gruppen zueinander wie z.B. 2,2-Dimethylpropan-1,3-diisocyanat. Im Falle cycloaliphatischer Polyisocyanate weist der, direkt mit den zur Bildung der erfindungsgemäßen polycyclischen Diiminooxadiazinonstruktur in 1,3-Stellung verknüpfte Cyclus im allgemeinen 4 bis 8, vorzugsweise 5 oder 6, besonders bevorzugt 6 Kohlenstoffatome auf und kann beliebige inerte Substituenten, insbesondere Alkylgruppen, vorzugsweise solche mit 1 bis 4 Kohlenstoffatomen aufweisen. Besonders bevorzugt sind solche 1,3-Diisocyanatocyclohexane, die in 2- und/oder in 4-Position einen oder mehrere C1-C20-Alkylsubstituenten aufweisen. Geeignete 1,3-Diisocyanatocycloalkane sind beispielsweise, 1,3-Diisocyanatocyclopentan, 1,3-Diisocyanatocyclohexan, 1,3-Diisocyanatocyclooctan, 1,3-Diisocyanato-2-methylcyclohexan, 1,3-Diisocyanato-4-methylcyclohexan sowie beliebige technische Gemische der beiden letztgenannten Diisocyanate, 1,3-Diisocyanato-2-isopropylcyclohexan, 1,3-Diisocyanato-4-isopropylcyclohexan, 1,3-Diisocyanato-2,4-dimethylcyclohexan, 1,3-Diisocyanato-2,4-diethylcyclohexan,1,3-Diisocyanato-2,4-diethyl-6-methylcyclohexan, 1,3-Diisocyanato-2-methyl-4,6-diethylcyclohexan sowie beliebige technische Gemische der beiden letztgenannten Diisocyanate oder 1,3-Diisocyanato-2,4,6-triisopropylcyclohexan sowie 1,3-Diisocyanato-2,4,6-tributylcyclohexan.

Es ist belanglos, nach welchen Verfahren die vorstehend genannten Isocyanate generiert werden, d.h. mit oder ohne Verwendung von Phosgen. Hierbei ist jedoch die Phosgenierung der entsprechenden Amine in der Gasphase besonders bevorzugt, da auf diese Weise reinere Produkte in höheren Ausbeuten als bei der Phosgenierung in flüssiger Phase resultieren (vgl. EP 0 676 392 A1 und EP 0 764 633 A1). Alternativ sind die vorstehend genannten Diisocyanate auch nach phosgenfreien Lehrbuchmethoden zugänglich, wie z. B. dem Curtius-Abbau entsprechend substituierter Carbonsäureazide wobei letztere nicht in Substanz isoliert werden müssen sondern in situ beispielsweise aus Carbonsäuren sowie Carbonsäurederivaten und Aziden verschiedenster Struktur zugänglich sind.

Die Umsetzung der vorstehend genannten 1,3-Diisocyanate zu den erfindungsgemäßen polycyclischen Diiminooxadiazinonen kann in Substanz oder in Verdünnung mit gegenüber Isocyanaten inerten Lösemitteln erfolgen. In einer weiteren bevorzugten Ausführungsform des erfindungsgemäßen Verfahrens wird die Umsetzung in Gegenwart mindestens eines Lösungsmittels durchgeführt. Dies ist bevorzugt, um das Ausmaß intermolekularer Reaktionen unter Bildung höhermolekularer Isocyanat-Folgeprodukte, d.h. von Verbindungen die aus mehr als 2 Monomereinheiten aufgebaut sind, zurück zu drängen. Wird unter Verdünnung gearbeitet, ist die Verwendung von Lösungsmitteln, die bei dem angewandten Druck oberhalb 150 °C sieden, bevorzugt, aber nicht Bedingung.

Diese Umsetzungen werden typischerweise katalysiert geführt, wobei als Katalysatoren eine ganze Reihe Isocyanat-aktiver Verbindungen zum Einsatz kommen können, die allgemein für die sog. Isocyanatmodifizierung zum Einsatz gelangen und beispielsweise in H. J. Laas et al., J. Prakt. Chem. 1994, 336, 185 ff beschrieben werden. Als Isocyanatmodifizierung wird dabei die Oligo- bzw. Polymerisierung von Isocyanaten insbesondere unter Bildung von höhermolekularen Oligomerengemischen mit Uretdion- ("Dimer"), Isocyanurat- ("Trimer") und/oder Iminooxadiazindionstrukturen ("asymmetrisches Trimer") im Molekülgerüst bezeichnet, die in der vorliegenden Schrift explizit **nicht** Zielverbindungen sind, deren anteilige Bildung neben den erfindungsgemäßen polycyclischen Diiminooxadiazinonen aber durchaus für viele übliche Einsatzgebiete der erfindungsgemäßen polycyclischen Diiminooxadiazinone tolerabel ist.

Es war sehr überraschend und für den Fachmann nicht zu erwarten, dass bei der erfindungsgemäßen Umsetzung spezieller Diisocyanate in denen die beiden NCO-Gruppen in 1,3-Stellung zueinander angeordnet sind, nicht (hauptsächlich) diese allgemein bekannten, höhermolekularen, modifizierten Polyisocyanate entstehen, sondern die Isocyanatgruppen-freien erfindungsgemäßen polycyclischen Diiminooxadiazinone, diskrete Moleküle eines bisher völlig unbekannten Strukturtyps.

In einer weiteren bevorzugten Ausführungsform des erfindungsgemäßen Verfahrens erfolgt die Cyclisierung in Anwesenheit mindestens eines Katalysators.

Hierbei ist es weiter bevorzugt, dass der Katalysator ein Phosphan oder ein Phosphanoxid ist, bevorzugt eine Verbindung ist, die mindestens ein Phosphoratom in einem 5-gliedrigen Ring aufweist und besonders bevorzugt ein Phospholanoxid oder ein Phospholenoxid ist.

Wenn das Verfahren in der vorstehend genannten, bevorzugten Ausführungsform umfassend die Schritte i) und ii) durchgeführt wird, ist der Katalysator zumindest in Schritt i) anwesend.

Als Katalysatoren für die erfindungsgemäße Umsetzung werden bevorzugt phosphororganische Verbindungen wie Phosphane und Phosphanoxide verwendet. Die Reaktion kann dabei auch so geführt werden, dass die für die Bildung der mindestens bicyclischen Iminooxadiazindione aus den zugrunde liegenden 1,3-Diisocyanaten gut geeigneten tertiären Phosphane nach im wesentlichen abgeschlossener Bildung ersterer zu den entsprechenden P-Oxiden oxidiert werden und so die anschließende Bildung der erfindungsgemäßen polycyclischen Diiminooxadiazinone mit mindestens einem zusätzlichen annelierten Ring gegenüber den mindestens bicyclischen Iminooxadiazindionen katalysieren.

Beispielhaft genannt seien (ab "propyl" alle Isomeren): Triethylphosphan, Tripropylphosphane, Tributylphosphane, Tripentylphosphane, Trihexylphosphane, Cyclopentyl-dimethylphosphan, Cyclopentyl-diethylphosphan, Cyclopentyl-dipropylphosphane, Cyclopentyl-dibutylphosphane, Cyclopentyl-dihexylphosphane, Cyclopentyl-dioctylphosphane, Dicyclopentyl-methylphosphan, Dicyclopentyl-ethylphosphan, Dicyclopentyl-n-propylphosphane, Dicyclopentyl-butylphosphane, Dicyclopentyl-hexylphosphane, Dicyclopentyl-octylphosphane, Tricyclopentylphosphan, Cyclohexyl-dimethylphosphan, Cyclohexyl-diethylphosphan, Cyclohexyl-dipropylphosphane, Cyclohexyl-dibutylphosphane, Cyclohexyl-dihexylphosphane, Cyclohexyl-dioctylphosphane, Dicyclohexyl-methylphosphan, Dicyclohexyl-ethylphosphan, Dicyclohexylpropylphosphane, Dicyclohexylbutylphosphane, Dicyclohexylhexylphosphane, Dicyclohexyloctylphosphane, Tricyclohexylphosphan sowie die P-Oxide der vorstehend genannten Verbindungen.

Weiterhin können auch Verbindungen als Katalysatoren zum Einsatz kommen, bei denen das Phosphoratom Teil eines n-gliedrigen Ringes ist, der seinerseits auch ggf. sp²-hybrididisierte Kohlenstoffatome aufweisen kann, mit n als natürliche Zahl, die bevorzugt 5 oder 6 ist. Insbesondere die P-Oxide dieser Fünfringspezies sind bereits aus der US 2 853 473 als hervorragende Carbodiimidisierungskatalysatoren bekannt. Beispielhaft seien genannt: 1-Methyl-1-phospholanoxid, 1-Methyl-1-phosphol-2-enoxid, 1-Methyl-1-phosphol-3-enoxid, 1,3-Dimethyl-1-phosphol-2-enoxid, 1,3- Dimethyl-1-phosphol- 3-enoxid, 1,3,4-Trimethyl-1-phosphol-2-enoxid, 1,3,4-Trimethyl-1-phosphol-3-enoxid, 1-Ethyl-1-phospholanoxid, 1-Ethyl-1-phosphol-2-enoxid, 1-Ethyl-1-phosphol-3-enoxid, 1-Ethyl-3-methyl-1-phosphol-2-enoxid, 1-Ethyl-3-methyl-1-phosphol-3-enoxid, 1-Ethyl-3,4-dimethyl-1-phosphol-2-enoxid, 1-Ethyl-3,4-dimethyl-1-phosphol-3-enoxid, 1-Phenyl-1-phospholanoxid, 1-Phenyl-1-phosphol-2-enoxid, 1-Phenyl-1-phosphol-3-enoxid, 1-Phenyl-3-methyl-1-phosphol-2-enoxid, 1-Phenyl-3-methyl-1-phosphol-3-enoxid, 1-Phenyl-3,4-dimethyl-1-phosphol-2-enoxid, 1-Phenyl-3,4-dimethyl-1-phosphol-3-enoxid.

Insbesondere die Phosphane sind ebenfalls gute Katalysatoren für die Bildung der mindestens bicyclischen Iminooxadiazindione aus den zugrundeliegenden 1,3-Diisocyanaten. Diese mindestens bicyclischen Iminooxadiazindione können als Precursoren für die erfindungsgemäßen polycyclischen Diiminooxadiazinone aufgefasst werden. So gelingt die Decarboxylierung ersterer zu letzteren auch auf rein thermischem Wege in Abwesenheit von Katalysatoren. Daher ist ebenfalls eine Ausführungsform des erfindungsgemäßen Verfahrens bevorzugt, bei der die Umsetzung in Schritt ii) thermisch induziert erfolgt.

Die Reaktion kann nach einem beliebig gewählten Umsetzungsgrad der monomeren Verbindung(en), beispielsweise durch Zugabe eines Katalysatorgiftes, durch thermische Desaktivierung des Katalysators oder durch einfaches Abkühlen des Reaktionsgemisches abgestoppt werden. Im letzteren Fall sollte bei der Katalysatorwahl bevorzugt darauf geachtet werden, dass der Katalysator beim Durchlaufen des für die 1-Nylonbildung kritischen Temperaturbereiches keine ausreichende (Rest)aktivität mehr besitzt oder dass dieser Temperaturbereich hinreichend schnell durchfahren wird ("Abschrecken") so dass ggf. vorhandene Anteile an nicht umgesetzten Monomeren nicht oder nur in einem, für die Applikation des jeweiligen Produktes, tolerierbaren Ausmaß zu cyclopolymerem 1-Nylon abreagieren. Führt man die Reaktion bis zum im Wesentlichen vollständigen Verbrauch der 1,3-Diisocyanate, ist mit derartigen Problemen nicht zu rechnen. Andernfalls kann anschließend vom verbliebenen, nicht umgesetzten Monomeren nach einer Methode des Standes der Technik wie z.B. Destillation, Dünnschichtdestillation oder Extraktion abgetrennt werden.

Die erfindungsgemäßen polycyclischen Diiminooxadiazinone können, ggf. im Gemisch mit anderen Isocyanatfolgeprodukten, durch die üblichen Verfahren des Standes der Technik isoliert werden, wie z.B. Dünnschichtdestillation, Extraktion, Kristallisation oder Molekulardestillation und fallen dabei als farblose oder schwach gefärbte Flüssigkeiten oder Feststoffe an. Letztere weisen, abhängig von den eingesetzten Isocyanat(gemisch)en, einen Schmelzbereich von ca. 30-250 °C auf.

Die erfindungsgemäßen polycyclischen Diiminooxadiazinone sind äußerst wertvolle Rohstoffe, die sowohl als Zwischenprodukt zur Herstellung bzw. Formulierung von Wirkstoffen als auch für die Verwendung im Kunststoff- und Lacksektor geeignet sind.

In letzterem Applikationsfeld können sie, rein oder in Verbindung mit anderen Isocyanatderivaten des Standes der Technik, wie Uretdion-, Biuret-, Allophanat-, Isocyanurat-, Urethan- sowie Carbodiimid-Polyisocyanaten, deren freie NCO-Gruppen ggf. mit typischen Blockierungsmitteln desaktiviert wurden, eingesetzt werden.

Somit ist die Verwendung der erfindungsgemäßen polycyclischen Diiminooxadiazinone als Komponente oder Zwischenprodukt zur Herstellung von Polyurethankunststoffen, geschäumten Polyurethankunststoffen, Lacken und Beschichtungsmitteln ein weiterer Gegenstand der vorliegenden Erfindung.

Vorliegend umfasst die Definition "Polyurethan" auch die strukturanalogen Polyharnstoffe, welche sich durch die Umsetzung von NCO-Gruppen mit dem Fachmann bekannten Polyaminen herstellen lassen, wobei sich die "Polyurethane" im engeren Sinne durch die Umsetzung von NCO-Gruppen mit Polyhydroxyverbindungen herstellen lassen.

Der besondere Vorteil der erfindungsgemäßen polycyclischen Diiminooxadiazinone ist zum einen darin zu sehen, dass sie, selbst bei längerer thermischer Belastung, keine Rückspalttendenz in die zugrundeliegenden monomeren Diisocyanate aufweisen, jedoch andererseits eine ausreichend hohe Reaktivität gegenüber vielen Verbindungen, die Zerevitinov-aktiven Wasserstoff enthalten, besitzen.

Ein weiterer Gegenstand der vorliegenden Erfindung ist eine Zusammensetzung enthaltend mindestens ein erfindungsgemäßes polycyclisches Diiminooxadiazinon und mindestens eine Verbindung, die eine oder mehrere Zerevitinoff-aktive Wasserstoffatome aufweist.

Die resultierenden Kunststoffe und Beschichtungen entsprechen in ihrer chemischen Natur weitgehend denen, die auf Basis Biuret- sowie Allophanatgruppen enthaltender (Lack)rohstoffe (cyclo)aliphatischer Diisocyanate erhalten werden und sind somit außerordentlich hochwertige Produkte mit dem für die genannten, bewährten Systeme des Standes der Technik typischen Eigenschaftsprofil, ohne jedoch die bereits angesprochenen Nachteile aufzuweisen.

Ebenfalls Gegenstand der vorliegenden Erfindung ist Verwendung einer erfindungsgemäßen Zusammensetzung zur Herstellung eines Verbundkörpers als auch Verbundkörper enthaltend eine erfindungsgemäße Zusammensetzung.

Neben den erfindungsgemäßen Verfahrensprodukten und den gegebenenfalls mitverwendeten weiteren Bindemittelkomponenten und Lacklösungsmitteln oder Lacklösungsmittelgemischen wie beispielsweise Toluol, Xylol, Cyclohexan, Chlorbenzol, Butylacetat, Ethylacetat, Ethylglykolacetat, Methoxypropylacetat, Aceton, Testbenzin, höher substituierte Aromaten (Solventnaphtha®, Solvesso®, Shellsol®, Isopar®, Nappar®, Diasol®) können in den Beschichtungen auch weitere Hilfs- und Zusatzmittel verwendet werden, wie z.B. Benetzungsmittel, Verlaufsmittel, Hautverhinderungsmittel, Antischaummittel, Mattierungsmittel, viskositätsregulierende Stoffe, Pigmente, Farbstoffe, UV-Absorber und Stabilisatoren gegen thermische und oxidative Einflüsse.

Die Beschichtungsmittel auf Basis der erfindungsgemäßen polycyclischen Diiminooxadiazinone können zur Beschichtung einer Vielzahl von Materialien dienen, wie z.B. Holz, Kunststoff, Leder, Metall, Papier, Beton , Mauerwerk, Keramik und Textil.

Die nachfolgenden Beispiele und Vergleichsbeispiele sollen die Erfindung näher erläutern, ohne sie jedoch darauf einzuschränken.

Alle Prozentangaben und ppm-Angaben sind, soweit nicht anders vermerkt, auf das Gewicht bezogen.

Alle Reaktionen wurden unter einer Stickstoffatmosphäre durchgeführt.

Mol-% Angaben wurden NMR-spektroskopisch ermittelt und beziehen sich immer, so nicht anders ausgewiesen, auf die Summe der NCO-Folgeprodukte. Die Messungen erfolgten auf dem Gerät DRX 700 der Fa. Bruker an ca. 1 %igen (¹H-NMR) bzw. ca. 50 %igen (¹³C-NMR) Proben in trockenem C₆D₆ bei 700 MHz (¹H-NMR) bzw. 176 MHz (¹³C-NMR) Messfrequenz. Als Referenz für die ppm-Skale wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung.

Die verwendeten Diisocyanate sind Produkte der Covestro Deutschland AG, D-51365 Leverkusen, alle anderen kommerziell zugänglichen Chemikalien wurden von der Fa. Aldrich, D-82018 Taufkirchen, bezogen. 1-Methyl-2-phospholen-1-oxid und 1-Methyl-3-phospholen-1-oxid, CAS-Nr. 872-45-7 und 930-38-1 (etwa äquimolares Isomerengemisch, im weiteren Text MPO) sind Produkte der Fa. Clariant AG, Muttenz, CH.

### Beispiel 1 Thermische Cyclisierung von mindestens bicyclischen Iminooxadiazindionen

Durch Reaktion einer jeweils 10%igen Lösung von
a) 1,3-Diisocyanatopropan
b) 1,3-Diisocyanato-3-methyl-butan bzw.
c) 1,3-Diisocyanatocyclohexan (cis/trans-Isomerengemisch)
in o-Dichlorbenzol bei 120°C mit ca. 10 mol-% (bezogen auf das jeweilige Diisocyanat) Tri-n-butyl-phosphan wurden die jeweiligen bicyclischen Iminooxadiazindione gewonnen, durch Vakuumdestillation/sublimation gereinigt und mittels NMR sowie GC-MS charakterisiert.

Bei b) entstand überraschenderweise mit hoher Selektivität hauptsächlich ein Isomer (weitere wurden in sehr geringer Konzentration mittels GC-MS im rohen Reaktionsgemisch detektiert) dem durch röntgen-kristallografische Untersuchungen die Struktur mit tertiär gebundener freier NCO-Gruppe und zwei Methylgruppen am nachbarständig zur Iminofunktion lokalisierten C-Atom zugeordnet werden konnte. Bei c) reagierte lediglich das cis-Isomer, das trans-Isomer wurde mit dem überschüssigen o-Dichlorbenzol destillativ als Vorlauf separiert.

Die so erhaltenen Produkte wurden 5 h bei 220°C erhitzt und nach Erkalten analysiert.

Die Identität des aus 1a) gewonnenen, einfachsten Derivates der erfindungsgemäßen Verbindungsklasse mit R¹ bis R¹² = H in Formel I wurde nach Sublimation im Vakuum und Umkristallisation mittels NMR und röntgen-kristallografischer Untersuchungen zweifelsfrei identifiziert. In der rohen Reaktionsmischung wurde mittels NMR und GC-MS das Vorliegen des Isomers der Struktur II (in deutlich geringerer Konzentration als I) sichergestellt.

Auch bei 1b und 1c werden hauptsächlich die Isomere der allg. Formel I gebildet und mittels Vakuumsublimation und anschließendes Umkristallisieren in reiner Form erhalten. In den rohen Reaktionsmischungen wurden allerdings mittels NMR und GC-MS ebenfalls die Isomeren der Struktur II (in deutlich geringerer Konzentration als I) detektiert.

### Beispiel 2 Katalytische Bildung aus den monomeren Diisocyanaten

Durch Reaktion einer jeweils 10%igen Lösung von
a) 1,3-Diisocyanatopropan
b) 1,3-Diisocyanato-3-methyl-butan bzw.
c) 1,3-Diisocyanatocyclohexan (cis/trans-Isomerengemisch)
in o-Dichlorbenzol bei 180°C mit ca. 5 mol-% (bezogen auf das jeweilige Diisocyanat) MPO wurden die jeweiligen polycyclischen Diiminooxadiazinone gewonnen, durch Vakuumdestillation/-sublimation gereinigt und mittels NMR sowie GC-MS charakterisiert. Die so erhaltenen Produkte unterscheiden sich nicht von denen, die gemäß Bsp. 1 isoliert wurden. Die Gesamtausbeute (bezogen auf das primär eingesetzte 1,3-Diisocyanat) ist allerdings signifikant höher als bei 1.

### Beispiel 3 Reaktion mit Zerevitinoff-aktives H tragenden Substanzen

Das gemäß Beispiel 2a) aus 1,3-Diisocyanatopropan erhaltene, tricyclische Diiminooxadiazinon wurde in überschüssigem 1-Butanol bzw. 1-Nonanthiol gelöst und 3-4 h bei 100 °C gerührt. Es fand in beiden Fällen vollständige Reaktion statt. Im 13C-NMR des erhaltenen Reaktionsgemisches waren die für das Edukt charakteristischen Signale (im Intensitätsverhätnis 2 : 1) bei 140,7 und 146,4 vollständig abwesend. Stattdessen erschienen je drei Signale im Tieffeldbereich bei 155,2, 153,9 und 149,6 ppm (Umsetzung mit 1-Butanol) bzw. 155,7, 153,0 und 151,9 ppm (Umsetzung mit 1-Nonanthiol), die charakteristisch für O-Alkylisoharnstoffe bzw. S-Alkylisothioharnstoffe sind.

Diese Modellreaktion belegt, dass die erfindungsgemäßen tricyclischen Diiminooxadiazinone als Isocyanatgruppen-freie Vernetzer für die Polyurethanchemie geeignet sind, da bei Wahl eine Di- bzw. Poly(thi)ols in Analogie zu den in Beispiel 3 gezeigten Reaktionen hochmolekulare Produkte zugänglich sind.

## Patentansprüche

1. Polycyclische Diiminooxadiazinone der Formel I und/oder der Formel II, 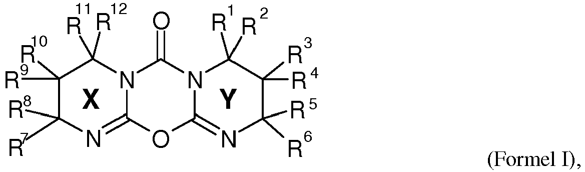 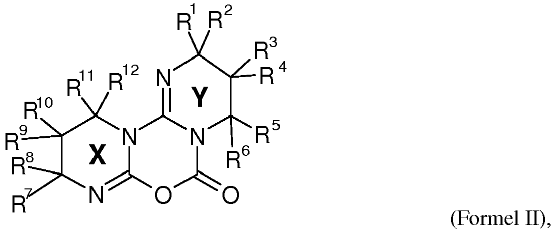 in welchen jeweils
R¹ bis R¹² unabhängig voneinander für Wasserstoff, einen gesättigten oder ungesättigten, linearen oder verzweigten, aliphatischen oder cycloaliphatischen organischen Rest mit 1 bis 20 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 20 Kohlenstoffatomen, der unsubstituiert oder mit gegenüber Isocyanatgruppen inerten Resten substituiert ist, stehen, wobei zwei oder mehrere der oben genannten Reste R¹ bis R⁶ jeweils miteinander und unter Einbeziehung eines, zweier oder dreier Kohlenstoffatome des annelierten Ringsegmentes Y weitere Ringe bilden können und/oder zwei oder mehrere der oben genannten Reste R⁷ bis R¹² jeweils miteinander und unter Einbeziehung eines, zweier oder dreier Kohlenstoffatome des annelierten Ringsegmentes X weitere Ringe bilden können.

2. Polycyclische Diiminooxadiazinone gemäß Anspruch 1, **dadurch gekennzeichnet, dass**
R¹ bis R¹² unabhängig voneinander für Wasserstoff, einen gesättigten oder ungesättigten, linearen oder verzweigten, aliphatischen oder cycloaliphatischen organischen Rest mit 1 bis 10 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 12 Kohlenstoffatomen, der unsubstituiert oder mit gegenüber Isocyanatgruppen inerten Resten substituiert ist, stehen, wobei zwei oder mehrere der oben genannten Reste R¹ bis R⁶ jeweils miteinander und unter Einbeziehung eines, zweier oder dreier Kohlenstoffatome des annelierten Ringsegmentes Y weitere Ringe bilden können und/oder zwei oder mehrere der oben genannten Reste R⁷ bis R¹² jeweils miteinander und unter Einbeziehung eines, zweier oder dreier Kohlenstoffatome des annelierten Ringsegmentes X weitere Ringe bilden können.

3. Polycyclische Diiminooxadiazinone gemäß Anspruch 1 oder 2, **dadurch gekennzeichnet, dass** R¹ bis R¹² unabhängig voneinander für Wasserstoff oder C₁-C₁₀-Alkyl(en) stehen.

4. Polycyclische Diiminooxadiazinone gemäß einem der Ansprüche 1 bis 3, **dadurch gekennzeichnet, dass** die gegenüber Isocyanatgruppen inerten Heteroatome Sauerstoff, Schwefel, tertiärer Stickstoff und/oder quartäres Silizium, wobei die Substituenten am Stickstoff und Silizium gegenüber Isocyanatgruppen inert sind, sind.

5. Polycyclische Diiminooxadiazinone gemäß einem der Ansprüche 1 bis 4, **dadurch gekennzeichnet, dass** diese Verbindungen der Formel III und/oder Verbindungen der Formel IV umfassen, 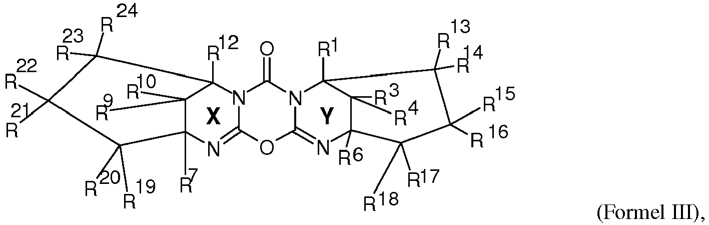 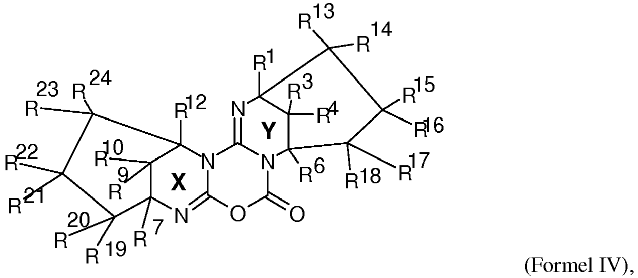 in welchen jeweils
R¹, R³, R⁴, R⁶, R⁷, R⁹, R¹⁰ und R¹² unabhängig voneinander für Wasserstoff, einen gesättigten oder ungesättigten, linearen oder verzweigten, aliphatischen oder cycloaliphatischen organischen Rest mit 1 bis 20 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 20 Kohlenstoffatomen, der unsubstituiert oder mit gegenüber Isocyanatgruppen inerten Resten substituiert ist, stehen und
R¹³ bis R²⁴ unabhängig voneinander für Wasserstoff, einen gesättigten oder ungesättigten, linearen oder verzweigten, aliphatischen oder cycloaliphatischen organischen Rest mit 1 bis 20 Kohlenstoffatomen, der unsubstituiert oder mit gegenüber Isocyanatgruppen inerten Resten substituiert ist, und/oder gegenüber Isocyanatgruppen inerte Heteroatome in der Kette aufweist, oder für einen aromatischen organischen Rest mit 6 bis 20 Kohlenstoffatomen, der unsubstituiert oder mit gegenüber Isocyanatgruppen inerten Resten substituiert ist, stehen,
wobei zwei oder mehrere der oben genannten Reste R¹, R³, R⁴, R⁶, R¹³ bis R¹⁸ jeweils miteinander weitere Ringe bilden können und/oder zwei oder mehrere der oben genannten Reste R⁷, R⁹, R¹⁰, R¹², R¹⁹ bis R²⁴ jeweils miteinander weitere Ringe bilden können.

6. Verfahren zur Herstellung eines polycyclischen Diiminooxadiazinons gemäß einem der Ansprüche 1 bis 5, **dadurch gekennzeichnet, dass** mindestens zwei Diisocyanate, bei denen die beiden NCO-Gruppen in 1,3-Position zueinander stehen, unter CO₂-Abspaltung cyclisiert werden.

7. Verfahren gemäß Anspruch 6, **dadurch gekennzeichnet, dass** die Diisocyanate in einem ersten Schritt i) zu einem polycyclischen Iminooxadiazindion cyclisiert werden, welches
a) isoliert wird und in einem weiteren Schritt ii) zu dem polycyclischen Diiminooxadiazinon umgesetzt wird oder
b) ohne Isolierung in einem weiteren Schritt ii) direkt weiter zu dem polycyclischen Diiminooxadiazinon umgesetzt wird.

8. Verfahren gemäß Anspruch 6 oder 7, **dadurch gekennzeichnet, dass** die Cyclisierung in Anwesenheit mindestens eines Katalysators erfolgt.

9. Verfahren gemäß Anspruch 8, **dadurch gekennzeichnet, dass** der Katalysator ein Phosphan oder ein Phosphanoxid ist.

10. Verfahren gemäß einem der Ansprüche 7 bis 9, **dadurch gekennzeichnet, dass** die Umsetzung in Schritt ii) thermisch induziert erfolgt.

11. Verfahren gemäß einem der Ansprüche 6 bis 10, **dadurch gekennzeichnet, dass** die Umsetzung in Gegenwart mindestens eines Lösungsmittels durchgeführt wird.

12. Verwendung der polycyclischen Diiminooxadiazinone gemäß einem der Ansprüche 1 bis 5 als Komponente oder Zwischenprodukt zur Herstellung von Polyurethankunststoffen, geschäumten Polyurethankunststoffen, Lacken und Beschichtungsmitteln.

13. Zusammensetzung enthaltend mindestens ein polycyclisches Diiminooxadiazinon gemäß einem der Ansprüche 1 bis 5 und mindestens eine Verbindung die eine oder mehrere Zerevitinoff-aktive Wasserstoffatome aufweist.

14. Verwendung einer Zusammensetzung gemäß Anspruch 13 zur Herstellung eines Verbundkörpers.

15. Verbundkörper enthaltend eine Zusammensetzung gemäß Anspruch 13.

## Claims

1. Polycyclic diiminooxadiazinones of the formula I and/or of the formula II, 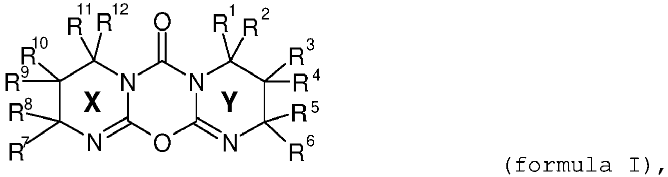 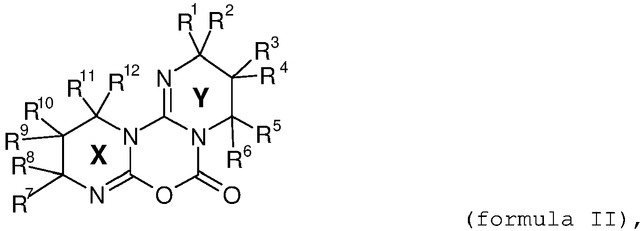 in which in each case
R¹ to R¹² are each independently hydrogen, a saturated or unsaturated, linear or branched, aliphatic or cycloaliphatic organic radical having 1 to 20 carbon atoms, which is unsubstituted or is substituted by radicals inert to isocyanate groups, and/or comprises heteroatoms inert to isocyanate groups in the chain, or are an aromatic organic radical having 6 to 20 carbon atoms, which is unsubstituted or is substituted by radicals inert to isocyanate groups, wherein two or more of the aforementioned radicals R¹ to R⁶ may form further rings, in each case with one another and incorporating one, two or three carbon atoms of the fused ring segment Y, and/or two or more of the aforementioned radicals R⁷ to R¹² may form further rings, in each case with one another and incorporating one, two or three carbon atoms of the fused ring segment X.

2. Polycyclic diiminooxadiazinones according to Claim 1, **characterized in that**
R¹ to R¹² are each independently hydrogen, a saturated or unsaturated, linear or branched, aliphatic or cycloaliphatic organic radical having 1 to 10 carbon atoms, which is unsubstituted or is substituted by radicals inert to isocyanate groups, and/or comprises heteroatoms inert to isocyanate groups in the chain, or are an aromatic organic radical having 6 to 12 carbon atoms, which is unsubstituted or is substituted by radicals inert to isocyanate groups, wherein two or more of the aforementioned radicals R¹ to R⁶ may form further rings, in each case with one another and incorporating one, two or three carbon atoms of the fused ring segment Y, and/or two or more of the aforementioned radicals R⁷ to R¹² may form further rings, in each case with one another and incorporating one, two or three carbon atoms of the fused ring segment X.

3. Polycyclic diiminooxadiazinones according to Claim 1 or 2, **characterized in that** R¹ to R¹² are each independently hydrogen or C₁-C₁₀-alkyl(ene).

4. Polycyclic diiminooxadiazinones according to any of Claims 1 to 3, **characterized in that** the heteroatoms inert to isocyanate groups are oxygen, sulfur, tertiary nitrogen and/or quaternary silicon, wherein the substituents on the nitrogen and silicon are inert to isocyanate groups.

5. Polycyclic diiminooxadiazinones according to any of Claims 1 to 4, **characterized in that** said compounds include compounds of the formula III and/or compounds of the formula IV, 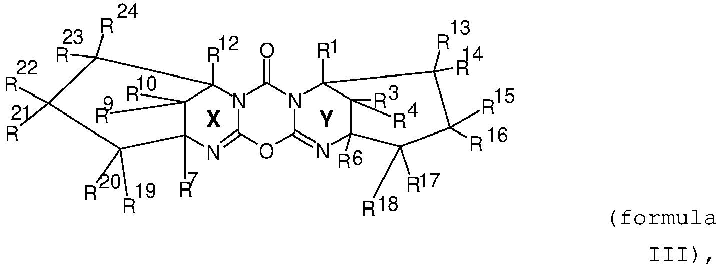 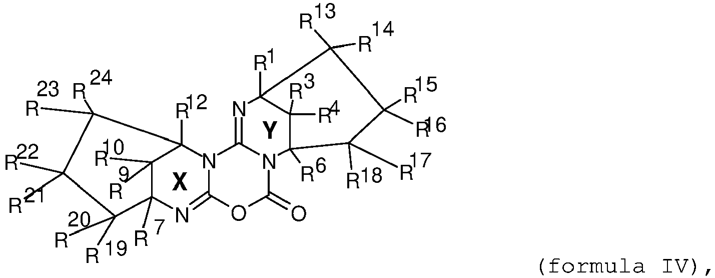 in which in each case
R¹, R³, R⁴, R⁶, R⁷, R⁹, R¹⁰ and R¹² are each independently hydrogen, a saturated or unsaturated, linear or branched, aliphatic or cycloaliphatic organic radical having 1 to 20 carbon atoms, which is unsubstituted or is substituted by radicals inert to isocyanate groups, and/or comprises heteroatoms inert to isocyanate groups in the chain, or are an aromatic organic radical having 6 to 20 carbon atoms, which is unsubstituted or is substituted by radicals inert to isocyanate groups, and
R¹³ to R²⁴ are each independently hydrogen, a saturated or unsaturated, linear or branched, aliphatic or cycloaliphatic organic radical having 1 to 20 carbon atoms, which is unsubstituted or is substituted by radicals inert to isocyanate groups, and/or comprises heteroatoms inert to isocyanate groups in the chain, or are an aromatic organic radical having 6 to 20 carbon atoms, which is unsubstituted or is substituted by radicals inert to isocyanate groups,
wherein two or more of the aforementioned radicals R¹, R³, R⁴, R⁶, R¹³ to R¹⁸ may form further rings in each case with one another and/or two or more of the aforementioned radicals R⁷, R⁹, R¹⁰, R¹², R¹⁹ to R²⁴ may form further rings in each case with one another.

6. Method for preparing a polycyclic diiminooxadiazinone according to any of Claims 1 to 5, **characterized in that** at least two diisocyanates, in which the two NCO groups are in a 1,3-position to each other, are cyclized with elimination of CO₂.

7. Method according to Claim 6, **characterized in that** the diisocyanates are cyclized in a first step i) to give a polycyclic iminooxadiazinedione, which
a) is isolated and is converted to the polycyclic diiminooxadiazinone in a further step ii) or
b) is further converted without isolation directly to the polycyclic diiminooxadiazinone in a further step ii).

8. Method according to Claim 6 or 7, **characterized in that** the cyclization is effected in the presence of at least one catalyst.

9. Method according to Claim 8, **characterized in that** the catalyst is a phosphane or a phosphane oxide.

10. Method according to any of Claims 7 to 9, **characterized in that** the reaction in step ii) is thermally induced.

11. Method according to any of Claims 6 to 10, **characterized in that** the reaction is carried out in the presence of at least one solvent.

12. Use of the polycyclic diiminooxadiazinones according to any of Claims 1 to 5 as a component or intermediate for producing polyurethane plastics, foamed polyurethane plastics, lacquers and coating compositions.

13. Composition comprising at least one polycyclic diiminooxadiazinone according to any of Claims 1 to 5 and at least one compound comprising one or more Zerevitinoff-active hydrogen atoms.

14. Use of a composition according to Claim 13 for producing a composite.

15. Composite comprising a composition according to Claim 13.

## Revendications

1. Diiminooxadiazinones polycycliques de la formule I et/ou de la formule II, 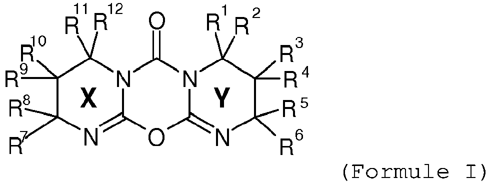 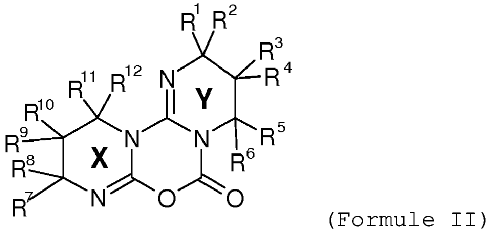 dans lesquelles à chaque fois
R¹ à R¹² représentent indépendamment les uns des autres l'hydrogène, un radical organique aliphatique ou cycloaliphatique, saturé ou insaturé, linéaire ou ramifié, de 1 à 20 atomes de carbone, qui est non substitué ou substitué avec des radicaux inertes vis-à-vis des groupes isocyanate, et/ou qui comprend des hétéroatomes inertes vis-à-vis des groupes isocyanate dans la chaîne, ou un radical organique aromatique de 6 à 20 atomes de carbone, qui est non substitué ou substitué avec des radicaux inertes vis-à-vis des groupes isocyanate,
deux ou davantage des radicaux R¹ à R⁶ susmentionnés pouvant former des cycles supplémentaires à chaque fois les uns avec les autres et avec inclusion d'un, de deux ou de trois atomes de carbone du segment de cycle annelé, et/ou deux ou davantage des radicaux R⁷ à R¹² susmentionnés pouvant former des cycles supplémentaires à chaque fois les uns avec les autres et avec inclusion d'un, de deux ou de trois atomes de carbone du segment de cycle annelé.

2. Diiminooxadiazinones polycycliques selon la revendication 1, **caractérisées en ce que** R¹ à R¹² représentent indépendamment les uns des autres l'hydrogène, un radical organique aliphatique ou cycloaliphatique, saturé ou insaturé, linéaire ou ramifié, de 1 à 10 atomes de carbone, qui est non substitué ou substitué avec des radicaux inertes vis-à-vis des groupes isocyanate, et/ou qui comprend des hétéroatomes inertes vis-à-vis des groupes isocyanate dans la chaîne, ou un radical organique aromatique de 6 à 12 atomes de carbone, qui est non substitué ou substitué avec des radicaux inertes vis-à-vis des groupes isocyanate,
deux ou davantage des radicaux R¹ à R⁶ susmentionnés pouvant former des cycles supplémentaires à chaque fois les uns avec les autres et avec inclusion d'un, de deux ou de trois atomes de carbone du segment de cycle annelé, et/ou deux ou davantage des radicaux R⁷ à R¹² susmentionnés pouvant former des cycles supplémentaires à chaque fois les uns avec les autres et avec inclusion d'un, de deux ou de trois atomes de carbone du segment de cycle annelé.

3. Diiminooxadiazinones polycycliques selon la revendication 1 ou 2, **caractérisées en ce que** R¹ à R¹² représentent indépendamment les uns des autres l'hydrogène ou un alkyl(ène) en C₁-C₁₀.

4. Diiminooxadiazinones polycycliques selon l'une quelconque des revendications 1 à 3, **caractérisées en ce que** les hétéroatomes inertes vis-à-vis des groupes isocyanate sont l'oxygène, le soufre, l'azote tertiaire et/ou le silicium quaternaire, les substituants sur l'azote et le silicium étant inertes vis-à-vis des groupes isocyanate.

5. Diiminooxadiazinones polycycliques selon l'une quelconque des revendications 1 à 4, **caractérisées en ce que** celles-ci comprennent des composés de la formule III et/ou des composés de la formule IV, 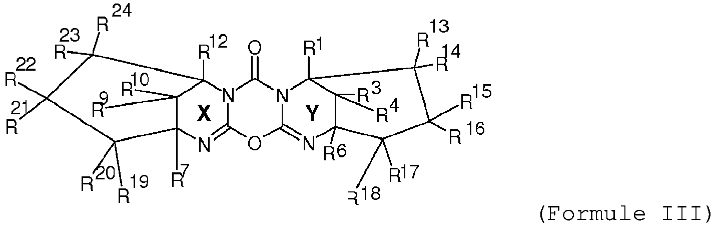 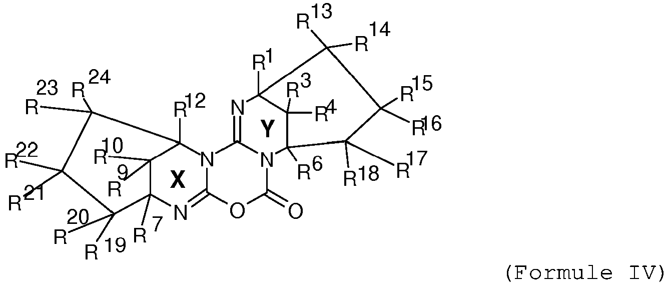 dans lesquelles à chaque fois
R¹, R³, R⁴, R⁶, R⁷, R⁹, R¹⁰ et R¹² représentent indépendamment les uns des autres l'hydrogène, un radical organique aliphatique ou cycloaliphatique, saturé ou insaturé, linéaire ou ramifié, de 1 à 20 atomes de carbone, qui est non substitué ou substitué avec des radicaux inertes vis-à-vis des groupes isocyanate, et/ou qui comprend des hétéroatomes inertes vis-à-vis des groupes isocyanate dans la chaîne, ou un radical organique aromatique de 6 à 20 atomes de carbone, qui est non substitué ou substitué avec des radicaux inertes vis-à-vis des groupes isocyanate, et
R¹³ à R²⁴ représentent indépendamment les uns des autres l'hydrogène, un radical organique aliphatique ou cycloaliphatique, saturé ou insaturé, linéaire ou ramifié, de 1 à 20 atomes de carbone, qui est non substitué ou substitué avec des radicaux inertes vis-à-vis des groupes isocyanate, et/ou qui comprend des hétéroatomes inertes vis-à-vis des groupes isocyanate dans la chaîne, ou un radical organique aromatique de 6 à 20 atomes de carbone, qui est non substitué ou substitué avec des radicaux inertes vis-à-vis des groupes isocyanate,
deux ou davantage des radicaux R¹, R³, R⁴, R⁶, R¹³ à R¹⁸ susmentionnés pouvant former des cycles supplémentaires à chaque fois les uns avec les autres, et/ou deux ou davantage des radicaux R⁷, R⁹, R¹⁰, R¹², R¹⁹ à R²⁴ susmentionnés pouvant former des cycles supplémentaires à chaque fois les uns avec les autres.

6. Procédé de fabrication d'une diiminooxadiazinone polycyclique selon l'une quelconque des revendications 1 à 5, **caractérisé en ce qu'**au moins deux diisocyanates, dans lesquels les deux groupes NCO se situent en position 1,3 l'un par rapport à l'autre, sont cyclisés avec clivage de CO₂.

7. Procédé selon la revendication 6, **caractérisé en ce que** les diisocyanates sont cyclisés lors d'une première étape i) en une iminooxadiazinedione polycyclique, qui
a) est isolée et, lors d'une étape ii) ultérieure, mise en réaction pour former la diiminoxadiazinone polycyclique, ou
b) mise en réaction directement lors d'une étape ii) ultérieure sans isolement pour former la diiminoxadiazinone polycyclique.

8. Procédé selon la revendication 6 ou 7, **caractérisé en ce que** la cyclisation a lieu en présence d'au moins un catalyseur.

9. Procédé selon la revendication 8, **caractérisé en ce que** le catalyseur est un phosphane ou un oxyde de phosphane.

10. Procédé selon l'une quelconque des revendications 7 à 9, **caractérisé en ce que** la réaction à l'étape ii) a lieu avec induction thermique.

11. Procédé selon l'une quelconque des revendications 6 à 10, **caractérisé en ce que** la réaction est réalisée en présence d'au moins un solvant.

12. Utilisation des diiminooxadiazinones polycycliques selon l'une quelconque des revendications 1 à 5 en tant que composant ou produit intermédiaire pour la fabrication de matières plastiques à base de polyuréthane, de matières plastiques à base de polyuréthane moussées, de vernis et d'agents de revêtement.

13. Composition contenant au moins une diiminooxadiazinone polycyclique selon l'une quelconque des revendications 1 à 5 et au moins un composé qui comprend un ou plusieurs atomes d'hydrogène actifs selon Zerevitinoff.

14. Utilisation d'une composition selon la revendication 13 pour la fabrication d'un corps composite.

15. Corps composite contenant une composition selon la revendication 13.
